Europäisches Patentamt

European Patent Office

Office européen des brevets

(19)

(11) Veröffentlichungsnummer: **0 181 992**
**B1**

(12) **EUROPÄISCHE PATENTSCHRIFT**

(45) Veröffentlichungstag der Patentschrift: **27.06.90**

(51) Int. Cl.⁵: **G 09 B 23/32**

(21) Anmeldenummer: **85102825.8**

(22) Anmeldetag: **12.03.85**

(54) Endoprothesen-Gelenkkomponente für Lehr-, Forschungs- und Anschauungszwecke.

(30) Priorität: **19.11.84 DE 8433918 u**

(43) Veröffentlichungstag der Anmeldung:
**28.05.86 Patentblatt 86/22**

(45) Bekanntmachung des Hinweises auf die
Patenterteilung:
**27.06.90 Patentblatt 90/26**

(84) Benannte Vertragsstaaten:
**CH DE FR GB LI SE**

(73) Patentinhaber: **Draenert, Klaus,
Dr.med.Dr.med.habil.
Gabriel-Max-Strasse 3
D-8000 München 90 (DE)**

(72) Erfinder: **Draenert, Klaus, Dr.med.Dr.med.habil.
Gabriel-Max-Strasse 3
D-8000 München 90 (DE)**

(74) Vertreter: **Vossius & Partner
Siebertstrasse 4 P.O. Box 86 07 67
D-8000 München 86 (DE)**

(56) Entgegenhaltungen:
**WO-A-82/01954**

**KUNSTSTOFFE, Band 72, Nr. 10, Oktober 1982,
Seiten 647-651, München; M. UNGETHÜM,
"Kunststoffe in der Knochenchirurgie"**

## Beschreibung

Mit der Einführung des Gelenkersatzes in die Orthopädie und Chirurgie des Bewegungsapparates wurden die Mediziner mit technischen und operationstechnischen Problemen konfrontiert, auf die sie durch das Hochschulstudium nicht vorbereitet werden.

Operationsverfahren, der Grundlage die Einführung derartiger neuer Technologien in die Medizin bilden, haben daher nur eine Chance, erfolgreich praktiziert zu werden, sofern Gefahren, Fehlplanungen, Fehler bei der Durchführung und Fehler in der Vor- und Nachbehandlung gelehrt, aufgezeigt und begründet werden und auch weiter Gegenstand einer anwendungsnahen Grundlagenforschung sind.

Die kritische Auswertung der Fehlergebnisse künstlicher Hüftgelenksimplantate aufgrund des bei Prothesenwechseloperationen gewonnenen histologischen Materiales ließ erkennen, daß die Hauptursache der Prothesenlockerung die Fehlimplantation der Prothesenkomponenten war.

Die Auswertung von makroskopischen und mikroskopisch-histologischen Befunden von Gelenkimplantaten, die zum Teil 20 Jahre implantiert waren und Leichen entnommen werden konnten, zeigte darüber hinaus, daß die Zementiertechniken und ihre Ergebnisse sehr stark differierten und oft zum großen Teil für Fehlergebnisse verantwortlich gemacht werden konnten.

Fehlermöglichkeiten beim Einsetzen einer Prothese liegen beispielsweise in der schlechten bzw. fehlerhaften Vorbereitung des Prothesenlagers. die Verbesserung der Implantationstechnik hat hier zum Ziel, ganz ähnlich wie bei Zahnimplantaten, ein möglichst sauberes und nach Möglichkeit sogar weitgehend trockenes Implantatlager zu schaffen.

Die Kontrolle des Markhöhlenabschlusses, die Penetration des Knochenzementes, die Verankerung beispielsweise im proximalen Femur, die Position der Prothesenkomponente, das Vermeiden des Schaffens von Spaltbildungen währen der Aushärtung der Knochenzemente und das Entfernen überschüssigen Zementes während und nach dem Aushärten der Zementkomponenten sind nur einige der Kriterien, die vom Operateur beherrscht und beurteilt werden müssen.

Von ganz vorrangiger Bedeutung zur Verbesserung der Zementiertechnik ist auch die Beurteilung und das Ausnützen des Druckaufbaues in der Zementspritze während der Applikation und währen des Aushärtens der Knochenzemente. Hierzu gehören die Probleme der Luftkomprimierung während der Zementapplikation in die Markhöhle, der Luft- und Knochenmarksembolie, die dabei auftreten kann, sowie das vasovagale Reflexverhalten beim Druckaufbau in der Markhöhle. Alle diese Phänomene müssen in systematischen Grundlagenforschungsstudien untersucht und erarbeitet werden.

Die modernen Techniken der Frakturbehandlung und des Gelenkersatzes werden in Operationskursen gelehrt, zu denen in erster Linie praktizierende Chirurgen der Orthopädie und der Traumatologie eingeladen werden. In diesen Operationskursen geht es vornehmlich darum, die Vorbereitung des knöchernen Lagers, die Verankerungstechnik der Prothesenkomponenten und die Position der Prothesenkomponenten im Knochen zu lehren und auf die Fehlermöglichkeiten bei diesen Verfahren hinzuweisen.

In der Prothetik stößt die Beurteilung des Operationsergebnisses in derartigen Kursen jedoch auf die Schwierigkeit, daß die in den Knochen versenkten Implantate nur dann exakt beurteilt werden können, wenn der Knochen mitsamt dem Implantat aufgeschnitten wird. Da die meisten Prothesenkomponenten aus einem sehr widerstandsfähigen Material, wie Metall, Keramik oder hochfestem Kunststoff hergestellt sind, ist dies aufgrund der hochkomplizierten Trennverfahren nicht möglich.

Auch in der Forschung und im Labor ist es mit den bekannten Prothesen aus wirtschaftlichen und verfahrenstechnischen Gründen nicht möglich, größere Serien von Implantationen zur systematischen Erforschung der Operationstechnik und möglicher Fehler durchzuführen.

Aus WO—A—82-01954 ist ein Fingermodell bekannt, das als Fingerendoprothese oder als Demonstrationsobjekt zur Erläuterung der Fingerbewegung und Verletzungen des Fingerbewegungsapparates verwendet werden soll. Die einzelnen Glieder des Fingermodells sind den jeweiligen Fingergliedern nachgebildet. Falls das Fingermodell als Prothese verwendet werden soll, bestehen die Glieder des Fingermodells aus einem harten Material, wie Keramik oder einem festen Kunststoff. Die Glieder des Fingermodells sind durch Gelenke aus einem elastischen Kunststoff verbunden.

Aus dem Aufsatz "Kunststoffe in der Knochenchirurgie", in Kunststoffe, Band 72, Nr. 10, Seiten 647—651 ist bekannt, daß verschiedene Kunststoffe, wie beispielsweise Silastik, grundsätzlich als Prothesenmaterial oder für Teile von Prothesen verwendet werden können.

Der Erfindung liegt die Aufgabe zugrunde, Endoprothesen-Gelenkkomponenten bereitzustellen, mit denen die vorstehenden Nachteile beseitigt und, auch in größeren Serien, in der Grundlagenforschung und in Operationskursen neue Operationstechniken gelehrt und weiter entwickelt und die Operationsergebnisse einfach und sicher beurteilt werden können.

Diese Aufgabe wird durch die Endoprothesen-Gelenkkomponente gemäß dem Patentansprüchen gelöst.

Die Erfindung geht dabe von dem Grundgedanken aus, Endoprothesen-Gelenkkomponenten aus einem leicht schneidbaren oder sägbaren Material zu entwickeln, die insbesondere als exakte Abgüsse oder nach Negativmodellen von üblichen, gängigen Gelenkkomponenten hergestellt werden können. Wenn derartige Gelenkkomponenten in Operationskursen implantiert werden, kann der Knochen mitsamt dem Implantat durch Aufsägen in einer einfachen Bandsäge schon währen des Kurses aufgeschnitten und danach das Operationsergebnis beurteilt werden, was entscheidenden didaktischen Wert hat; besonders geeignete Materialien hierfür sind Kunststoffe, wie spritzgegossene Kunststoffe oder Geißharze. Unter üblichen Gelenkkomponenten sind dabei nicht nur die derzeit üblichen Gelenkkomponenten zu verstehen, sondern auch zukünftig entwickelte und als Gelenkersatz operativ verwendbare Gelenkkomponenten oder Prothesen.

Da es bei der Applikation und Verankerungstechnik der Prothesenkomponenten nahezu ausschließlich auf den Volumeneffekt der Prothesen und nicht auf ihre mechanische Dauerwiderstandsfähigkeit ankommt, spielt die Stabilität des Werkstoffes der Prothesenkomponente hierbei nur eine untergeordnete Rolle. Im Gegensatz zu den Metallprothesen eignen sich die Kunststoffprothesen viel besser für die Kurse, da die in den Knochen implantierten Prothesen mit einer einfachen Bandsäge sehr schnell entlang ihrer medialen Naht in zwei Hälften zersägt werden können, so daß das Operationsergebnis, nämlich insbesondere die Ausbildung des Zementmantels, die Penetration des Zementes, das Abskinken des Zementes in die Markhöhle, die Applikation des die Markhöhle verschließenden Plugs, die Verankerung beispielsweise im proximalen Femur, die Position der Prothese, der Zementmantel um die Prothesenkomponente und dergl. exakt beurteilt und kritisch gewertet werden kann.

Die Schulungsprothesen aus Kunststoff haben darüber hinaus den Vorteil, daß sie in allen Farben hergestellt werden können, was den visuellen Eindruck und damit die Erinnerungsfähigkeit sehr verstärkt. Das Operationsergebnis läßt sich besonders eindrucksvoll darstellen, wenn sowohl der für das Implantat verwendete Kunststoff als auch der Zement farblich markiert ist, so daß sich auch Schichtungen des Zements oder das dynamische Verhalten von Fließvorgängen beurteilen lassen. Wenn Implantat und Zement leicht aufgetrennt werden können, läßt sich das Operationsergebnis einfach und in kurzer Zeit für die Teilnehmer des Kurses dokumentieren.

Bevorzugt sind Farbzusätze oder Farbaufträge, die beim Sägen nicht absplittern bzw. ein Splittern der Kunststoffe verhindern.

Die Anwendung der erfindungsgemäßen Gelenkkomponenten in Kursen ist auch insoweit vorteilhaft, da aufgrund der Wirtschaftlichkeit dieser Gelenkkomponenten jeder einzelne Teilnehmer eine Serie von Operationen durchführen kann und die Operationsergebnisse als Anschauungsmaterial in aufgearbeiteter Form mit nach Hause nehmen kann. Auf diese Weise können die Teilnehmer ihrerseits wiederum in kleineren und größeren Kreisen die Operationsergebnisse in Lehre und Fortbildung weitergeben. Damit läßt sich letztendlich das Ziel erreichen, die klinischen Kurzzeit-, vor allem aber auch die Langzeitergebnisse von Gelenkersatzoperationen zu verbessern.

Darüber hinaus sind die erfindungsgemäßen Gelenkkomponenten aufgrund ihres günstigen Preises auch für die Forschung ein Objekt, das es ermöglicht, größere Serien von Implantationen zur systematischen Erforschung der Operationstechnik und möglicher Fehler, die bei der Implantation von Gelenkkomponenten gemacht werden können, durchzuführen. Derartige Serienuntersuchungen waren bisher aufgrund des teuren Materials und der aufwendigen Aufarbeitungs- und Trennungstechniken nicht oder nur in geringem Maße durchführbar.

Die erfindungsgemäßen Endoprothesen-Gelenkkomponenten werden allen herkömmlichen, üblichen Gelenkkomponenten nachgebildet. Beim für die erfindungsgemäßen Gelenkkomponenten verwendeten Material kommt es einerseits darauf an, daß es mechanisch ausreichend fest und widerstandsfähig und präzise genug zu bearbeiten ist, um eine Simulation des Operationsergebnisses der bei tatsächlichen Operationen verwendeten Prothesen zu erzielen, andererseits sollte das Material mit herkömmlichen Sägen leicht trennbar sein.

Die Schalenprothesen werden vorteilhafterweise im Ausgußverfahren hergestellt. Die im Handel befindlichen Gießharze gewährleisten eine hohe Präzision dieser Ausgußmodelle und bieten darüber hinaus den Vorteil, daß die Prothesenkomponenten in einfacher Weise in verschiedenen Farben hergestellt werden können. Besonders bevorzugte Gießharze sind Epoxidharze, Polyesterharze, Acrylharze und Phenolharze.

Die Schaftprothesen werden vorteilhafterweise nach dem etwas teureren Spritzgußverfahren mit Herstellung einer Spritzgußform und der Serienproduktion in Spritzgußmaschinen hergestellt. Auf diese Weise gelingt es, mechanisch widerstandsfähige Schulungsprothesen herzustellen, die ebenfalls in allen Farben zur Verfügung gestellt werden können. Besonders bevorzugte, nach dem Spritzgußverfahren zu verarbeitende Kunststoffe sind Styrol-Butadien, Styrol-Acrylnitril, Polyvinylidenfluorid, Polyurethan, Polystyrol, Polyphenylensulfid, Polypropylen, Polyoxymethylen, Polymethylmethacrylat, Polyäthylen, Polycarbonat, Polybutylenterephthalat, Polyamid und Acrylnitril-Butadien-Styrol.

Die erfindungsgemäßen Endoprothesen-Gelenkkomponenten für Lehr-, Forschungs- und Anschauungszwecke können allen herkömmlichen, üblichen Prothesenkomponenten nachgebildet sein und entsprechen diesen in ihrer äußerlichen Form und Gestalt. Nachstehend werden einige herkömmliche Prothesenkomponenten anhand der Zeichnung näher erläutert. Die erfindungsgemäßen Gelenkkomponenten aus einem spritzgegossenen Kunststoff oder einem Gießharz entsprechen in ihrer äußeren Gestaltung diesen herkömmlichen Prothesenkomponenten. Es zeigen:

Figur 1 herkömmliche Schultergelenk- und Ellbogengelenkkomponenten,

Figur 2 herkömmliche Kniegelenkkomponenten in Form einer sogenannten "Schlittenprothese",

Figur 3 herkömmliche Kniegelenkkomponenten in Form eines Scharniergelenkes,

Figur 4 ein konventionelle Hüftgelenks-Totalprothese,

Figur 5 eine weitere Hüftgelenks-Totalprothese aus zwei Schalen,

Figur 6 herkömmliche Handgelenkkomponenten,

Figur 7 eine erfindungsgemäße, im Spritzgußverfahren hergestellte Standard-Müller-Prothese und

Figur 8 erfindungsgemäße, im Gießverfahren hergestellte Wagner-Schalen.

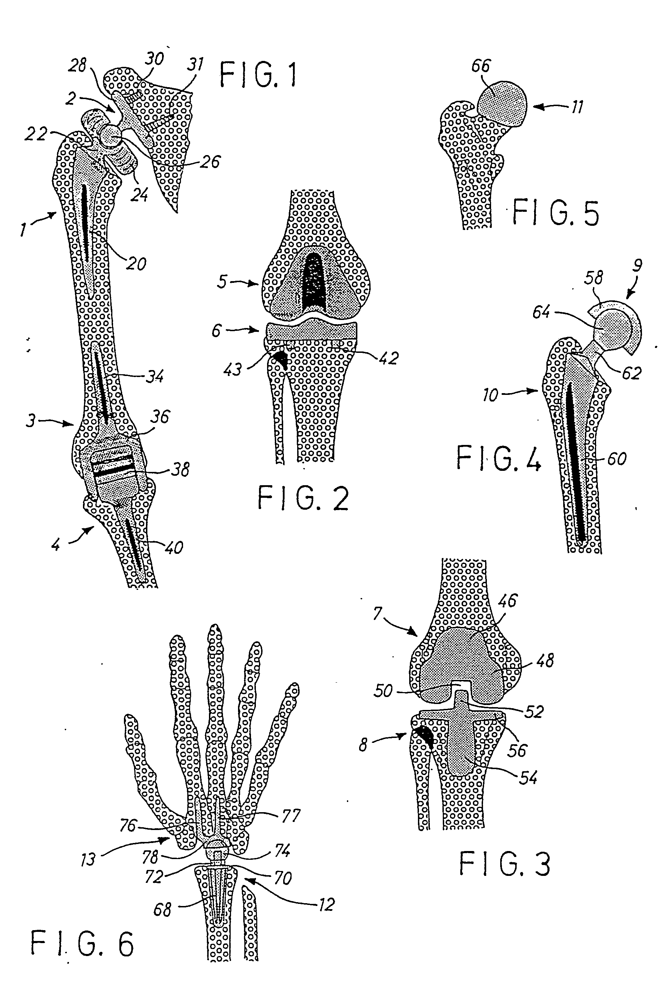

Figur 1 zeigt eine herkömmliche Schultergelenkkomponente 1 mit einem im Oberarmschaft versenkten, sich verjüngenden Schaftteil 20, einer sich daran anschließenden Verjüngung 22 und einer darauf aufgesetzten Platte oder Scheibe 24, die etwa mittig eine annähernd kugelförmige Ausnehmung aufweist. Die im Oberarmschaft versenkte Komponente 1 wirkt mit der Schultergelenkkomponente 2 zusammen, die eine in die Ausnehmung der Platte 24 eingreifende Kugel 26, einen verjüngten Mittelteil und eine Platte 28 aufweist, die mit vorzugsweise zwei Zapfen 30, 31 in der Schulter verankert ist. Erfindungsgemäß bestehen die Komponenten 1 und 2 aus einem spritzgegossenen Kunststoff oder einem Gießharz.

Neben den in Figur 1 dargestellten Schultergelenkkomponenten sind noch weitere, nicht dargestellte Schultergelenkkomponeten üblich. Die im Oberarmschaft versenkte Komponente dieser Schultergelenkprothese weist einen sich verjüngenden Schaftteil und einen darauf aufgesetzten, etwa halbkugelförmigen Kopf auf. Der Kopf ist meist etwas breiter als der Schaftteil, so daß am Übergang zwischen Kopf und Schaftteil ein Kragen ausgebildet wird, der auf dem Humerus aufsitzt. Die Im Oberarmschaft versenkte erste Komponente wirkt mit der zweiten Schultergelenkkomponente zusammen, die als schmale Pfanne oder Schale ausgebildet ist. Die Innenseite der Pfanne ist als Teil einer Kugelfläche ausgebildet und nimmt die kugelförmige Zirkumferenz des Gelenkkopfes der ersten Komponente auf. Die Pfanne der zweiten Komponente ist meist aus Metall oder einem sehr widerstandsfähigen Kunststoff hergestellt und in den Knochen verschraubt; erfindungsgemäß bestehen die erste und die zweite Komponente aus einem spritzgegossenen Kunststoff oder einem Gießharz.

Am Ellbogen werden üblicherweise Scharniergelenke verwendet. Die beiden Ellbogengelenkkomponenten 3 und 4 sind mit ihrem Schaftteil im Knochenmarksraum des Oberarmknochens bzw. der Elle versenkt. Der Radius mit seinem Gelenkende wird meist reseziert. Die im Oberarmknochen versenkte Komponente 3 weist am Ende des Schaftteiles 34 eine Verbreiterung 36 auf, die eine zentrale, etwa rechteckige Ausnehmung aufweist, die den entsprechend ausgebildeten Kopf 38 der in der Elle Versenkten Komponente 4 aufnimmt. Die Komponente 4 kann zwischen ihrem Schaftteil 40 und Kopf 38 einen umlaufenden Kragen oder Anschlag aufweisen, der auf der Elle aufsitzt.

Figur 2 zeigt eine Kniegelenk-Schlittenprothese vom Typ I. Die Kniegelenkkomponente 5 wird mit einem mehr oder weniger ausgedehnten Ersatz der Knorpelgelenkfläche im distalen Femur verankert. Sie kann verschraubt werden, meist wird sie jedoch nur durch zwei oder mehrere kurze Zapfen im Knochen verankert. Ihre Form ist der Form des distalen Femur nachgebildet. Die ihr gegenüberliegende Kniegelenkkomponente 6 ist in Form des Tibiaplateaus meist aus Kunststoff ausgebildet und passiv mit zwei oder mehreren Fortsätzen 42, 43 im Knochen verzapft.

Figure 3 zeigt den Type II eines herkömmlichen Kniegelenkersatzes, der, wie das Ellbogengelenk, als Scharniergelenk ausgebildet ist, das mit zwei Schäften jeweils oben und unten, vorzugsweise mit Knochenzement, im Knochen verankert wird. Der Schaftteil 46 der im Femur verankerten Komponente 7 weist am distalen Ende eine Verbreiterung 48 mit eine zentralen, etwa rechteckigen Ausnehmung 50 auf, die einen entsprechend geformten Vorsprung oder Kopf 52 der in der Tibia verankerten Komponente 8 aufnimmt.

Die Komponente 8 weist zwischen Schaft 54 und Kopf 52 am Tibiaplateau eine plattenförmige Verbreiterung 56 auf.

Die konventionelle Hüftgelenks-Totalprothese gemäß Figur 4 weist eine als Plastikpfanne 58 ausgebildete und mit Knochenzement oder einem, zwei oder mehreren Zapfen im Dach verankerte Komponente 9 auf. Im Fermurschaft ist eine als Schaftprothese mit Gelenkkopf ausgebildete Gelenkkomponente 10 in den Knochen versenkt. Der Schaft 60 der Komponente 10 ist gebogen oder gerade und zwischen dem Schaft und dem auf einer zylindrischen Fortsetzung 62 des Schaftes ruhenden, annähernd kugelförmigen Gelenkkopf 64 kann eine umlaufender Rand oder Kragen geformt sein, der auf dem Femur aufsitzt. Die Komponente 10 wird entweder mit Knochenzement oder selbstverblockend im Fermur fixiert; beim letztgenannten Typ ist kein umlaufender Rand oder Kragen ausgebildet.

Figur 5 zeigt einen anderen Type des totalen Hüftgelenkersatzes, der aus zwei Schalen besteht (double cup). Hierbei wird lediglich die knorpelige Gelenkfläche ersetzt. Am Femurkopf ist eine als Metall- oder Keramikschale 66 ausgebildete Gelenkkomponente 11 zementiert oder geschraubt, und im Bereich der Gelenkpfanne des Beckens ist ebenfalls eine (in Figur 5 nicht dargestellte) Kunststoffschale zementiert oder verschraubt, die die Komponente 11 aufnimmt.

Am Handgelenk sind die gleichen Gelenktypen wie am Ellbogen bekannt, wobei zwischen den beiden Gelenkkomponenten meist eine Kugel zwischengeschaltet ist. Gemäß Figur 6 weist die Handgelenkkomponente 12 einen im Radius verankerten Schaftteil 68 vorzugsweise mit einem kleinen Kragen 70 am distalen Ende der Elle, einen kurzen, vorzugsweise zylindrischen Mittelteil 72 und einen Kopf 74 auf. Der Kopf 74 ist nahezu kugelförmige ausgebildet und sitzt mit seinem abgeflachten Ende direkt auf dem Mittelteil 72 auf. Die Handgelenkkomponente 13 ist mit zwei oder mehreren Zapfen 76, 77 schaftförmig in der Handwurzel und Mittelhand verankert. Die beiden Zapfen 76, 77 sind etwa gabelförmig ausgebildet und durch eine Basis 78 die Handgelenkkomponente 13 miteinander verbunden. Die Basis 78 weist eine etwa zentrale Ausnehmung in Form eines Kugelabschnittes auf, die einen Teil des kugelförmigen Kopfes 74 der Händgelenkkomponente 12 aufnimmt, wobei die Höhe des Kugelabschnittes etwa die Hälfte bis zwei Drittel des Radius des Kopfes 74 beträgt. Die Ausnehmung ist etwa in Verlängerung eines der beiden Zapfen 77 in der Basis 78 ausgebildet.

Neben den in den Figuren 1 bis 6 gezeigten Gelenkkomponenten ist noch ein Gelenkersatz an den Fingergelenken und am Sprunggelenk bekannt.

Die Fingergelenkchen sind meist als Scharniergelenke mit beidseits im Knochenschaft verankerten Prothesenkomponenten ausgebildet.

Der Am Sprunggelenk herkömmliche Gelenkersatz entspricht in seiner Gestaltung der in Figure 2 gezeigten Kniegelenk-Schlittenprothese vom Typ I.

Sämtliche vorstehend erläuterten und alle weiteren, bekannten Gelenkkomponenten werden erfindungsgemäß aus einem leicht schmeidbaren oder sägbaren Material, wie einem spritzgegossenen Kunststoff oder einem Gießharz hergestellt und zu Lehr- und Forschungszwecke verwendet, wobei die vorstehend erläuterten Vorteile gegenüber den insbesondere aus Metall hergestellten herkömmlichen Gelenkkomponenten erzielt werden.

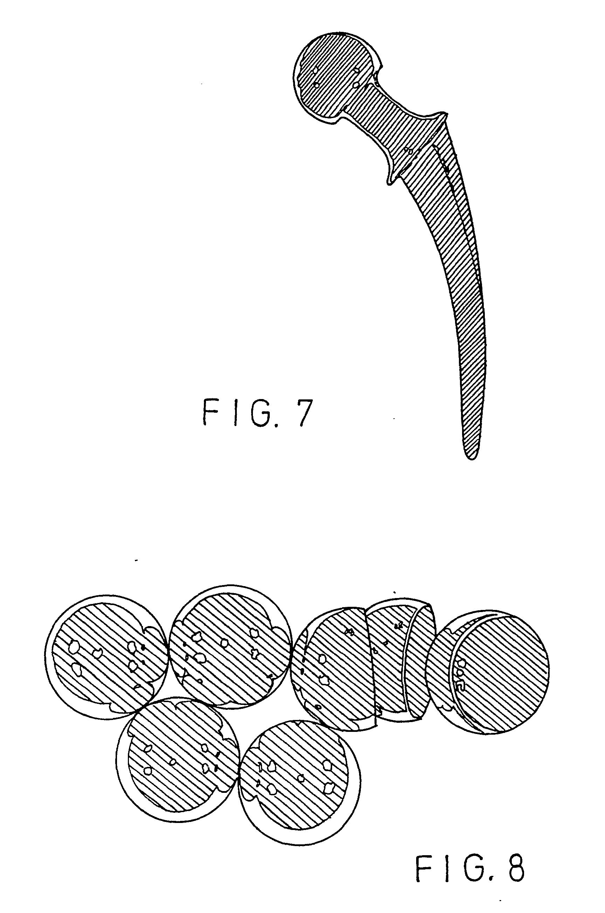

Die in Figur 7 dargestellte, im Spritzgußverfahren hergestellte Standard-Müller-Prothese entspricht der Gelenkkomponente 10 gemäß Figur 4, mit einem gebogenen, sich nach außen verjüngenden Schaft, einem bei der Implantation auf dem Femur aufsitzenden Kragen, einem etwa zylindrischen Mittelteil und einem nahezu kugelförmigen Gelenkkopf, wobei der Gelenkkopf zum Mittelteil hin abgeflacht ist.

Figur 8 zeigt erfindungsgemäße, nach dem Gießverfahren hergestellt Wagner-Schalen.

Die erfindungsgemäßen Schulungsprothesen, die in den Figuren 7 und 8 dargestellt sind bzw. die den in den Figuren 1 bis 6 dargestellten Prothesen nachgebildet sind, können beispielsweise auf folgenden Gebieten angewandt werden:

—Operationskurse für praktizierende Orthopäden und Chirurgen,

—Studentenunterricht an der Hochschule,

—Entwicklungslaboratorien der Herstellerfirmen,

—Grundlagenforschungsinstitute von Hochschulen und Entwicklungsinstituten,

—als Anschauungsmaterial für Fortbildungsunterricht für Studenten und Schwestern,

—Phantommodelle für die präoperative Planung,

—Phantommodelle für die makroskopische und mikroskopische Dokumentation der Marketing- und Werbeabteilungen,

—als Werbepräsent von Herstellerfirmen von Prothesen und Biomaterialien, wie Knochenzement.

Die erfindungsgemäßen Abguß- bzw. Spritzguß-Kunststoffprothesen weisen für Schulungs- und Forschungszwecke, insbesondere in Operationskursen in der Gelenkersatzchirurgie, gegenüber normalen Implantaten entschiedende Vorteile auf.

Die erfindungsgemäßen Endoprothesen-Gelenkkomponenten lassen sich kostengünstig herstellen und sind universell in Kursprogrammen und als Anschauungsmaterial sowie in der Modelltechnik verwendbar. Darüber hinaus bieten sie den Vorteil, daß sie mit allen gängigen Trennverfahren verarbeitet und zur Beurteilung der Operationsergebnisse aufgesägt werden können.

Zementierkurse mit Wertung der Ergebnisse werden praktisch erst durch die erfindungsgemäßen Endoprothesen-Gelenkkomponenten möglich. Für das Marketing und die Werbeabteilungen bieten sich neue Wege der Dokumentation und Instruktion für die Anwender und für Lehr- und Fortbildungsveranstaltungen.

Vorteilhafterweise sind die erfindungsgemäßigen Endoprothesen-Gelenkkomponenten auch in der systematischen Grundlagenforschung einzusetzten, da sie eine schnelle und kostengünstige Aufarbeitung des Materials erlauben. In systematischen Vergleichsstudien kann die Verankerung verschiedener Prothesentypen dargestellt werden, um die Möglichkeiten und Schwächen des Zementierergebnisses für den jeweiligen Prothesentyp festzuhalten.

**Patentansprüche**

1. Endoprothesen-Gelenkkomponente zur simulierten Implantation in Knochen für Lehr-, Forschungs- und Anschauungszwecke, wobei die äußere Forme und Gestaltung der Gelenkkomponente (1—13) üblichen Gelenkkomponenten entspricht und die Gelenkkomponente (1—13) aus einem leicht schneidbaren oder sägbaren Material besteht.

2. Gelenkkomponente nach Anspruch 1, dadurch gekennzeichnet, daß die Gelenkkomponente (1—13) aus einem spritzgegossenen Kunststoff oder aus einem Gießharz besteht.

3. Gelenkkomponente nach Anspruch 2, dadurch gekennzeichnet, daß der spritzgegossene Kunststoff Styrol-Butadien, Styrol-Acrylnitril, Polyvinylidenfluorid, Polyurethan, Polystyrol, Polyphenylsulfid, Polypropylen, Polyoxymethylen, Polymethylmethacrylat, Polyäthylen, Polycarbonat, Polybutylenterephthalat, Polyamid oder Acrylnitril-Butadien-Styrol und das Gießharz ein Epoxidharz, Polyesterharz, Acrylharz oder Phenolharz ist.

4. Gelenkkomponente nach einem der Ansprüche 1 bis 3, dadurch gekennzeichnet, daß sie farbig ist.

5. Gelenkkomponente nach einem der Ansprüche 1 bis 4, dadurch gekennzeichnet, daß die äußere Gestaltung der Gelenkkomponente (1—13) üblichen Schulter-, Ellbogen, Hüft, Knie-, Hand-, Finger, oder Sprunggelenkkomponenten nachgebildet ist.

6. Gelenkkomponente nach einem der Ansprüche 1 bis 5, dadurch gekennzeichnet, daß sie einer herkömmlichen Schultergelenkkomponente (1) nachgebildet ist und einen sich verjüngenden Schaftteil (20), eine Verjüngung (22) und eine Platte oder Scheibe (24) mit einer etwa mittigen, kugelförmigen Ausnehmung aufweist, oder daß sie einer herkömmlichen Schultergelenkkomponente (2) nachgebildet ist und einen kugelförmigen Kopf (26), einen verjüngten Mittelteil und eine Platte (28) mit zwei oder mehreren Zapfen (30, 31) aufweist, oder daß sie einer herkömmlichen Schultergelenkkomponente nachgebildet ist und einen sich verjüngenden Schaftteil und einen etwa halbkugelförmigen Kopf aufweist, oder daß sie einer kerkömmlichen Schultergelenkkomponente nachgebildet ist und eine schmale Pfanne oder Schale aufweist, deren Innenseite als Teil einer Kugelfläche ausgebildet ist.

7. Gelenkkomponente nach einem der Ansprüche 1 bis 5, dadurch gekennzeichnet, daß sie einer herkömmlichen Ellbogengelenkkomponente (3) nachgebildet ist und einen Schaftteil (34) mit einer Verbreiterung (36) an einem seiner Enden aufweist, die etwa mittig in Verlängerung des Schaftteils eine etwa quader-, scheibe- oder zungenförmige Ausnehmung aufweist, so daß der Querschnitt der Gelenkkomponente (3) etwa die Form einer Zange aufweist, oder daß sie einer herkömmlichen Ellbogengelenkkomponente (4) nachgebildet ist und einen Schaftteil (40), einen etwa quader- scheiben- oder zungenförmigen Kopf (38) und gegebenenfalls einen Kragen zwischen Schaftteil und kopf aufweist.

8. Gelenkkomponente nach einem der Ansprüche 1 bis 5, dadurch gekennzeichnet, daß sie die äußere Gestaltung einer Kniegelenkkomponente (5) in Form einer Schlittenprothese aufweist, die der Form des distalen Femurs nachgebildet ist, oder daß sie die äußere Gestaltung einer herkömmlichen Kniegelenkomponente (6) aufweist, die dem Tibiaplateau nachgebildet ist und zwei oder mehrere zapfenartige Fortsätze (42, 43) aufweist, oder daß sie einer herkömmlichen Kniegelenkkomponente (7) in Form eines Scharniergelenkes nachgebildet ist und einen sich verjüngenden Schaftteil (46) mit einer Verbreiterung (48) an einem seiner Enden aufweist, die etwa mittig in Verlängerung des Schaftteils eine etwa quader-, scheiben- oder gabelförmige Ausnehmung (50) aufweist, so daß der Querschnitt der Gelenkkomponente (7) etwa die Form einer Zunge aufweist, oder daß sie einer herkömmlichen Kniegelenkkomponente (8) in Form eines Scharniergelenkes nachgebildet ist und einen Schaftteil (54) mit einer plattenförmigen Verbreiterung (56) an einem seiner Enden aufweist, die etwa mittig in Verlängerung des Schaftteils einen etwa quader-, scheiben- oder gabelförmigen Vorsprung (52) aufweist.

9. Gelenkkomponente nach einem der Ansprüche 1 bis 5, dadurch gekennzeichnet, daß sie der in der Hüfte zu verankernden Komponente (9) einer konventionellen Hüftgelenk-Totalprothese (9, 10) nachgebildet ist und die Form einer Pfanne (58) mit etwa halbkugelförmiger Ausnehmung aufweist, wobei vorzugsweise mindestens ein Zapfen an der Außenseite der Pfanne (58) vorgesehen ist, oder daß sie der im Femur zu verankernden Komponente (10) einer konventionellen Hüftgelenks-Totalprothese (9, 10) in Form einer Standard-Prothese oder selbstverblockenden Prothese nachgebildet ist und einen gebogenen, sich verjüngenden Schaft (60), einen etwa zylinderförmigen Mittelteil (62) und einen annähernd kugelförmigen Kopf (64) aufweist, wobei vorzugsweise zwischen dem breiten Ende des Schaftes (60) und dem Mittelteil (62) ein umlaufender Rand oder Kragen angeordnet ist, oder daß sie die äußere Gestaltung einer herkömmlichen Hüftgelenkkomponente (11) in Form einer der Gestalt des Femurkopfes nachgebildeten Schale (66) aufweist.

10. Gelenkkomponente nach einem der Ansprüche 1 bis 5, dadurch gekennzeichnet, daß sie einer herkömmlichen, im Radius zu verankernden Handgelenkkomponente (12) nachgebildet ist und einen Schaftteil (68) mit einem kurzen Kragen (70), einen Mittelteil (72) und einen etwa kugelförmigen Kopf (74) aufweist, oder daß sie einer herkömmlichen, in der Handwurzel und Mittelhand zu verankernden Handgelenkkomponente (13) nachgebildet ist und einen Schaftteil mit vorzugsweise zwei oder mehreren Zapfen (76, 77) und eine Basis (78) mit einer Ausnehmung in Form eines Kugelabschnittes aufweist.

**Revendications**

1. Partie articulée pour endoprothèse en implantation osseuse simulée à des fins recherches, et de visualisation et dans le cadre d'un enseignement caractérisée en ce que la forme et la structure extérieures de la partie articulée (1 à 13) est conforme à la partie articulée normale, et la partie articulée (1 à 13) est constituée d'un matériau pouvant facilement être découpe ou seié.

2. Partie articulée selon la revendication 1, caractérisée en ce que la partie articulée (1 à 13) est constituée d'une matière plastique moulée par injection ou d'une résine moulée.

3. Partie articulée selon la revendication 2, caractérisée en ce que la matière plastique moulée par injection est du styrolène-butadiène, styrolène- acrylnitryle, fluorure de polyvinylidène, polyuréthane, polystyrolène, sulfure de polyphénylène, polypropylène, polyoxyméthylène, polyméthacrylate de méthyle, polyéthylène, polycarbonate, téréphthalate de polybutylène, polyamide ou styrène-butadiène-acrylnitrile, et en ce que la résine moulée est une résine époxy, une résine polyester, une résine acrylique ou une résine phénolique.

4. Partie articulée selon l'une quelconque des revendications 1 à 3, caractérisée en ce qu'elle est colorée.

5. Partie articulée selon l'une quelconque des revendications 1 à 4, caractérisée en ce que la structure extérieure de la partie articulée (1 à 13) reproduit la partie articulée de l'épaule, du coude, de la hanche, du genou, de la main, du doigt ou de l'articulation tibio-tarsienne.

6. Partie articulée selon l'une quelconque des revendications 1 à 5, caractérisée en ce qu'elle reproduit une partie d'épaule articulée normale (1), et en ce qu'elle comporte une partie de tige (20) allant en se rétrécissant, un rétrécissement (22) et une plaque ou un disque (24) comportant un creux sensiblement central de forme sphérique, ou en ce qu'elle reproduit une partie d'épaule articulée normale (2) et comporte une tête (26) de forme sphérique, une partie centrale rétrécie, et une plaque (28) comportant au moins deux goujons (30, 31), ou en ce qu'elle reproduit une partie d'épaule articulée normale et comporte une partie de tige allant en se rétrécissant et une tête de forme approximativement semi-sphérique, ou en ce qu'elle reproduit une partie d'épaule articulée normale et comporte une étroite cavité ou coquille dont une face interne forme une partie d'une surface sphérique.

7. Partie articulée selon l'une quelconque des revendications 1 à 5, caractérisée en ce qu'elle reproduit une partie de coude articulée normale (3) et comporte une partie de tige (34) présentant un élargissement (36) sur une de ses extrémités, laquelle comporte de manière approximativement centrale, en prolongement de la partie de tige, un creux en forme approximativement de parallélépipède rectangle, de disque ou de langue, de façon qu'une section transverale de la partie articulée (3) se présente approximativement sous la forme d'une pince, ou en ce qu'elle reproduit une partie de coude articulée normale (4) et comporte une partie de tige (40), une tête (38) en forme de parallépipède rectangle, de disque ou de langue, et, s'il y a lieu, une collerette placée entre la partie de tige et la tête.

8. Partie articulée selon l'une quelconque des revendications 1 à 5, caractérisée en ce qu'elle présente la structure extérieure d'une partie de genou articulée (5) sous la forme d'une prothèse-support, laquelle se présente sous la forme d'un fémur distal, ou en ce qu'elle présente la structure extérieure d'une partie de genou articulée normale (6) laquelle constitue une reproduction de plateau de tibia et comporte deux ou plus prolongements à effet d'emboîtement (42, 43), ou en ce qu'elle reproduit une partie de genou articulée normale (6), sous la forme d'une articulation à charnière et en ce qu'elle comporte une partie de tige (46) allent en ce rétrécissant, présentant un élargissement (48) sur une de ses extrémités, laquelle, approximativement au centre d'un prolongement de la partie de tige, comporte un creux (50) en forme approximativement de parallélépipède rectangle, de disque ou ayant une forme fourchue de façon qu'une section transversale de la partie articulée (7) présente approximativement la forme d'une langue, ou en ce qu'elle reproduit une partie de genou articulée normale (8) sous la forme d'une articulation à charnière et comporte une partie de tige (54) comprenant un élargissement (56) en forme de galette sur une de ses extrémités, laquelle présente approximativement au centre du prolongement de la partie de tige, une saillie (52) en forme de parallélépipède rectangle, de disque ou de forme fourchue.

9. Partie articulée selon l'une quelconque des revendications 1 à 5, caractérisée en ce qu'elle reproduit une partie (9) d'une prothèse coxofémorale conventionelle complète (9, 10) à ancrer à l'interieur de la hanche, et en ce qu'elle présente la forme d'une cavité, (58) comprenant un creux d'une forme approximativement demi-sphérique, par laquelle, de préférence est prévu au moins un amboîtement sur la face externe de la cavité (58), ou en ce qu'elle reproduit une partie (10) d'une prothèse coxofémorale conventionelle complète (9, 10) à ancrer dans le fémur, sous la forme d'une prothèse standard ou d'une prothèse à auto-blocage et en ce qu'elle comporte une tige (60) courbe allant en ce rétrécissant, une partie centrale (62) de forme approximativement cylindrique et une tête (64) de forme sensiblement sphérique, par laquelle est placée, de préférence entre l'extrémité aplatie de la tige (60) et la partie centrale (62), une bordure ou collerette circonférentielle ou en ce qu'elle présente la structure extérieure d'une partie de hanche articulée normale (11) en forme de coquille (66) reconstituée sous l'aspect d-une tête de fémur.

10. Partie articulée selon l'une quelconque des revendications 1 à 5, caractérisée en ce qu'elle reproduit une partie de main articulée normale (12) à ancrer dans le radius et en ce qu'elle comporte une partie de tige (68) comprenent une colerette courte (70), une partie centrale (72) et une tête (74) de forme approximativement sphérique ou en ce qu'elle comporte une partie de main articulée normale (13) à ancrer dans le carpe et dans le métacarpe et en ce qu'elle comporte une partie de tige comprenant de préférence deux ou plus emboîtements (76, 77) et un élément de base (78) présentant un creux en forme de calotte sphérique.

## Claims

1. An endoprosthesis joint component for simulated implantation in the bone for the purpose of teaching, research and demonstration, wherein the appearance of the joint component (1 to 13) corresponds to conventional joint components, and the joint component (1 to 13) is made of a material which is easy to cut or saw.

2. The joint component according to claim 1, wherein the joint component (1 to 13) is made of injection moulded plastic or of a casting resin.

3. The joint component according to claim 2, wherein the injection moulded plastic material is styrene butadiene, styrene acrylonitrile, polyvinylidene fluoride, polyurethane, polystyrene, polyphenylene sulfide, popypropylene, polyoxymethylene, polymethyl methacrylate, polyethylene, polycarbonate, polybutylene terephthalate, polyamide or acrylonitrile butadiene styrene and the casting resin is epoxy casting resin, polyester resin, acrylic resin or phenolic resin.

4. The joint component according to any of claims 1 to 3, wherein said component is coloured.

5. The joint component according to any of claims 1 to 4, wherein the appearance of the joint component (1 to 13), is a copy of conventional joint components of the shoulder, the elbow, the hip, the knee, the hand, the fingers or the ankle.

6. The joint component according to any of claims 1 to 5, wherein it is a copy of a conventional shoulder joint component (1) and exhibits a tapered stem portion (20), a tapering (22) and a plate or disc (24) having a spherical recess in its approximate centre, or wherein it is a copy of a conventional shoulder joint component (2) and exhibits a spherical head (26), a tapered central part and a plate (28) having two or more pins (30, 31) or wherein it is a copy of a conventional shoulder joint component and exhibits a tapered stem portion and an approximately semispherical head, or wherein it is a copy of a conventional shoulder joint component and exhibits a narrow socket or cup whose inside is shaped as part of a spherical surface.

7. The joint component according to any of claims 1 to 5, wherein it is a copy of a conventional elbow joint component (3) and exhibits a stem portion (34) having a broadening (36) at one of its ends which exhibits approximately in its centre and cuboid-shaped, disc-shaped or tongue-shaped recess and thus the cross-section of joint component (3) has the approximate shape of tongs, or wherein it is a copy of a conventional elbow joint component (4) and exhibits a stem portion (40), an approximately cuboid-shaped, disc-shaped or tongue-shaped head (38) as a continuation of the stem portion and, optionally, a collar between the stem portion and the head.

8. The joint component according to any of claims 1 to 5, wherein it exhibits the appearance of a knee joint component (5) in the shape of a sliding prosthesis, which prosthesis is a copy of the form of the distal femur, or wherein it exhibits the appearance of a conventional knee joint component (6) which is a copy of the tibial plateau and exhibits two or more pin-shaped protrusions (42, 43), or wherein it is a copy of a conventional knee joint component (7) in the shape of a hinged joint and exhibits a tapered stem portion (46) having a broadening (48) at one of its ends which exhibits in its approximate centre an approximately cuboid-shaped, disc-shaped or fork-shaped recess (50) as a continuation of the stem portion, and thus the cross-section of the joint component (7) is approximately tongue-shaped, or wherein it is a copy of a conventional knee joint component (8) in the shape of a hinged joint and exhibits a stem portion (54) having a plate-shaped broadening (56) at one of its ends which exhibits in its approximate centre an approximately cuboid-shaped, disc-shaped or fork-shaped projection (52) as a continuation of the stem portion.

9. The joint component according to any of claims 1 to 5, wherein it is a copy of the component (9) of a total hip prosthesis shaft for the hip joint (9, 10) to be anchored in the hip and exhibits the form of a socket (58) having an approximately semispherical recess, with preferably at least one pin being provided on the outer surface of the socket (58), or wherein it is a copy of the component (10) of a conventional total hip prosthesis (9, 10) to be locked in the femur, it being a Standard prosthesis or a self-locking prosthesis and exhibiting an arched, tapered stem (60), a roughly cylinder-shaped central part (62) and an approximately spherical head (64), it being preferable for a circumferential rim or collar to be arranged between the broad end of the stem (60) and the central part (62), or wherein it exhibits the appearance of a conventional hip joint component (11) in the form of a cup (66) which is a copy of the head of the femur.

10. The joint component according to any of claims 1 to 5, wherein it is a copy of a conventional hand joint component (12) to be anchored in the radius and exhibits a stem portion (68) having a short collar (70), a central part (72) and an approximately spherical head (74), or wherein it is a copy of a conventional hand joint component (13) to be anchored in the wrist or the metacarpus and exhibits a stem portion having preferably two or more pins (76, 77) and a base (78) having a recess in the form of a spherical section.

FIG. 1

FIG. 2

FIG. 3

FIG. 4

FIG. 5

FIG. 6

F I G. 7

F I G. 8